# EUROPÄISCHE PATENTANMELDUNG

(11) **EP 0 697 205 A1**
(43) Veröffentlichungstag der Anmeldung: **21.02.1996**
(21) Anmeldenummer: 95112794.3
(22) Anmeldetag: 14.08.1995
(51) Int. Cl.: A61J 15/00, A61M 25/10

(54) **Magensonde**

(30) Priorität: 17.08.1994 DE 9413272 U
(71) Anmelder: Roewer, Norbert, Dr., D-22529 Hamburg (DE); B. BRAUN MELSUNGEN AG, D-34212 Melsungen (DE)
(72) Erfinder: Roewer, Norbert, Prof. Dr., D-22529 Hamburg (DE); Klute, Volker, Dipl.-Ing., D-34212 Melsungen (DE)
(74) Vertreter: Glawe, Delfs, Moll & Partner

(57) **Zusammenfassung**

Eine Magensonde weist einen Saugschlauch (1) und einen Magenballon (7) auf, der durch Spannung des Saugschlauchs (1) in Anlage an der den Mageneingang (5) umgebenden Magenwand (12) zu halten ist. Der Magenballon ist mit einem Druckkontrollgerät verbunden, das zur Anzeige von mindestens zwei Druckstufen eingerichet ist, von denen die niedrigere dem bei ungespanntem Schlauch freigeblähten Zustand des Magenballons und die höhere dem gespannten Zustand des Saugschlauch zugeordnet ist. Der Magenballon (7) ist mit dem Saugschlauch an Stellen geringen gegenseitigen Abstands angebracht, so daß er im geblähten Zustand etwa Torusgestalt annimmt. Er ist in Längsrichtung des Schlauches elastisch nachgiebig.

## Beschreibung

Es ist eine Magensonde (EP-B 423234) mit einem Magenballon bekannt, der durch die Spannung des Saugschlauchs in abdichtender Anlage an der den Mageneingang umgebenden Magenwand zu halten ist. Der Abdichtungszustand wird dadurch aufrechterhalten, daß die Spannung des Saugschlauchs mittels eines Nasenstoppers aufrechterhalten wird. Der Magenballon ist mit einem Druckkontrollgerät verbunden, das es auf folgende Weise gestattet, die richtige Lage des Magenballons und die hinreichende Spannung des Saugschlauchs zu kontrollieren. Nach dem Legen der Sonde wird zunächst der im Magen befindliche Magenballon frei mittels auf eine erste, niedrige Druckstufe gebläht, während der Saugschlauch spannungslos ist. Danach wird der Saugschlauch gespannt, wodurch der Magenballon sich unter entsprechendem Druck an die Magenwand anlegt. Sein Innendruck steigt dabei mit zunehmender Schlauchspannung entsprechend dem Druck, mit dem der Ballon an der Magenwand anliegt. Wenn das Druckmeßgerät zeigt, daß eine zweite, höhere Druckstufe erreicht ist, von der man weiß, daß sie einem für die Abdichtung ausreichenden Anlagedruck entspricht, wird der erreichte Zustand mittels des Nasenstoppers fixiert.

Um den Mageninhalt gegenüber der Speiseröhre hinreichend abdichten zu können, ist ein gewisser Mindestanlagedruck erforderlich. Die Zugkraft am Schlauch ist gleich dem Produkt aus diesem Druck und der Anlagefläche, in der dieser Druck wirkt. Zur Schonung des Patienten ist man bestrebt, diese Kraft so gering wie möglich zu halten. Auch wünscht man eine hohe Elastizität der aus Saugschlauch und Magenballon bestehenden Anordnung, damit eine Änderung der Strecke, in der der Saugschlauch zwischen dem Magen und dem Nasenstopper gespannt ist, wie beispielsweise durch eine Änderung der Körper- oder Kopfhaltung des Patienten oder eine unbemerkte Verschiebung des Nasenstoppers zustande kommen kann, sich nicht oder nur wenig auf den zwischen dem Magenballon und der Magenwand herrschenden Anlagedruck auswirkt. Jedoch konnte die Zugkraft am Schlauch mit der bekannten Anordnung nicht wirksam gesenkt werden, weil einerseits der Anlagedruck für eine wirksame Abdichtung einen gewissen Mindestwert nicht unterschreiten darf und andererseits der Ballon so groß sein muß, daß er nicht ungewollt aus dem Mageneingang entweichten kann. Daraus ergibt sich eine beträchtliche Mindestgröße der im Schlauch herrschenden Zugkraft.

Die Erfindung erreicht eine Senkung dieser Kraft und eine Erhöhung der Elastizität dadurch, daß der Abstand zwischen den Befestigungsstellen, an denen die beiden Wände des Magenballons am Saugschlauch befestigt sind, wesentlich geringer gehalten wird als die Radialabmessung des Schlauchs, gemessen von seinem äußersten Umfang bis zur Schlauchoberfläche, wobei der Magenballon so ausgebildet wird, daß er in Längsrichtung des Schlauchs elastisch nachgiebig ist. Die genannten Maßverhältnisse sind dann zu messen, wenn der Ballon unter dem Anwendungsdruck gebläht ist. Zweckmäßigerweise ist der Abstand der Befestigungsstellen der Ballonwände nicht größer als zwei Drittel der genannten Radialabmessung des Ballons. Vorzugsweise liegt er in der Größenordnung von etwa der Hälfte dieses Werts. Der erfindungsgemäße Ballon wird auch dadurch charakterisiert, daß der Abstand der Befestigungsstellen geringer ist als die größte Ausdehnung des Ballons in Richtung parallel zu dem ihn durchsetzenden Schlauchabschnitt, wobei dieser Abstand vorzugsweise nicht größer ist als zwei Drittel dieser Ausdehnung. Unter den Befestigungsstellen sind diejenigen Stellen zu verstehen, an denen unter dem Balloninnendruck die den Ballon bildende Membran sich frei von dem Schlauch nach außen von diesem weg spannt.

Die Wirkung der erfindungsgemäßen Maßnahme besteht darin, daß der Ballon nicht die Erscheinungsform einer Kugel oder eines Ellipsoids, sondern etwa eines Torus hat. Dies hat einerseits für die Art der Anlage des Ballons an der Magenwand und andererseits für die Elastizität der Anordnung Konsequenzen. Zum einen liegt der Magenballon nicht mit vollem Druck vollflächig an der ihm zugewendeten, den Mageneingang umgebenden Magenwand an, sondern hauptsächlich in einer Ringfläche, die einen gewissen Radialabstand von der Schlauchmitte und der Mitte der Magenöffnung hat. Auch ist die radiale Breite dieser Ringfläche gering. Innerhalb des von der Ringfläche umschlossenen, zentrumsnäheren Bereich berührt der Ballon die Magenwand nicht oder nur unter einem geringeren Druck als in der Ringfläche. Dies schadet nicht, weil es ausreicht, wenn der dichtende Anlagedruck in der verhältnismäßig begrenzten Ringfläche erzielt wird. Die Schlauchspannung ist daher bei gleicher Abdichtungswirkung geringer als bei Verwendung eines kugelförmigen Ballons.

Zum anderen zeigt sich, daß der torusförmige Ballon eine erhebliche Elastizität in Schlauchlängsrichtung hat. Der Anlagedruck und der Balloninnendruck ändern sich nur wenig wenn der Ballon sich unter dem Zug des Saugschlauchs verformt. Dies hat den Vorteil, daß die Abdichtwirkung nicht so stark von einer gleichbleibenden Körper- und Kopfhaltung des Patienten abhängt wie bei der bekannten Magensonde.

Eine weiterer Vorteil der Erfindung besteht darin, daß der Ballon in geringerem Maße der Gefahr unterliegt, durch den Mageneingang herausgezogen zu werden. Er kann deshalb mit geringerem Durchmesser ausgestattet werden. Dadurch wird die Anlagefläche weiter verkleinert und der Schlauchzug herabgesetzt.

Aus der US-A 2 267 875 ist ein Katheter mit torusförmigem Halteballon bekannt, der im Gegensatz zu der Erfindung unelastisch sein soll. Die besonderen Vorteile, die dann erreicht werden, wenn ein torusförmiger Ballon mit elastischen Eigenschaften in dem besonderen Zusammenhang des unter Schlauchspannung abedichtenden Magenballons verwendet wird, lassen sich bei der Lektüre der genannten Schrift nicht erkennen.

Diese Schrift lehr auch den Aufbau des Ballons aus zwei im entspannten Zustand im wesentlichen ebenen, am Außenrand miteinander und am Innenrand mit dem Schlauch dicht verbundenen Folienstücken. Diese Anordnung hat im Zusammenhang der Erfindung den Vorteil, daß der Ringbereich des Ballons, der für die Abdichtung verwendet wird, im wesentlichen faltenfrei bleibt.

Die erfindungsgemäß erzielte Verringerung des im Saugschlauch herrschenden Zugs ist so erheblich, daß in vielen Fällen auf einen Nasenstopper zugunsten eines Speiseröhrenballons verzichtet werden kann. Dieser Vorschlag ist an sich bekannt aus der DE-A 24 12 553; jedoch hat er in die Praxis keinen Eingang finden können. In dem bekannten Fall wird ein kugelförmiger Magenballon verwendet, der nur mit verhältnismäßig hoher Schlauchspannung gegenüber der Magenwand abgedichtet werden kann. Ein Speiseröhrenballon vermag aber eine so hohe Schlauchspannung nicht zu halten, weil er wegen der Empfindlichkeit der Speiseröhre nur mit geringer Kraft an dieser anliegen darf und daher nur eine geringe Reibkraft erzeugt werden kann. Da somit die Schlauchspannung nur gering ist, kann der Magenballon keine hinreichende Abdichtwirkung erreichen. Dies war offenbar auch dem Urheber der bekannten Sonde klar, denn er schlägt vor, den Speiseröhrenballon zur zusätzlichen Abdichtung zu verwenden. Aber auch dies hält realistischer Nachprüfung nicht stand, weil wegen der Empfindlichkeit der Speiseröhre der durch den Speiseröhrenballon dort ausübbare Druck so gering ist, daß er keine hinreichende Abdichtwirkung ermöglicht.

Bekannt ist es auch (US-A 3 046 988), die Kombination eines Magen- und Speiseröhrenballons zum Verschließen von Blutungen aus oberflächlichen Blutgefäßen am Mageneingang und in der Speiseröhre zu verwenden. Da der Speiseröhrenballon den Magenballon nicht in der vorgesehenen Lage festhalten kann, ist ein Nasenstopper vorgesehen.

Die Haltewirkung des Speiseröhrenballons braucht nicht alleine auf der Reibung gegenüber der Speiseröhrenwandung zu beruhen. Vielmehr kann erfindungsgemäß vorgesehen sein, daß er sich zusätzlich oder überwiegend an der von dem unteren Speiseröhrenschließmuskel gebildeten Verengung abstützt. Der Abstand zwischen dem Magenballon und dem Speiseröhrenballon wird dann so bemessen, daß der Speiseröhrenballon die zur Abstützung erforderliche Lage dann aufweist, wenn der Saugschlauch die für die korrekte Lage des Magenballons erforderliche Spannung hat. Dabei wirkt sich günstig aus, daß der erfindungsgemäße Magenballon eine größere axiale Nachgiebigkeit aufweist als herkömmliche kugelige oder ellipsoidische Ballons, so daß er unterschiedliche anatomische Maßverhältnisse ausgleichen kann.

Die Erfindung wird im folgenden näher unter Bezugnahme auf die Zeichnung erläutert, die ein vorteilhaftes Ausführungsbeispiel veranschaulicht. Darin zeigen:

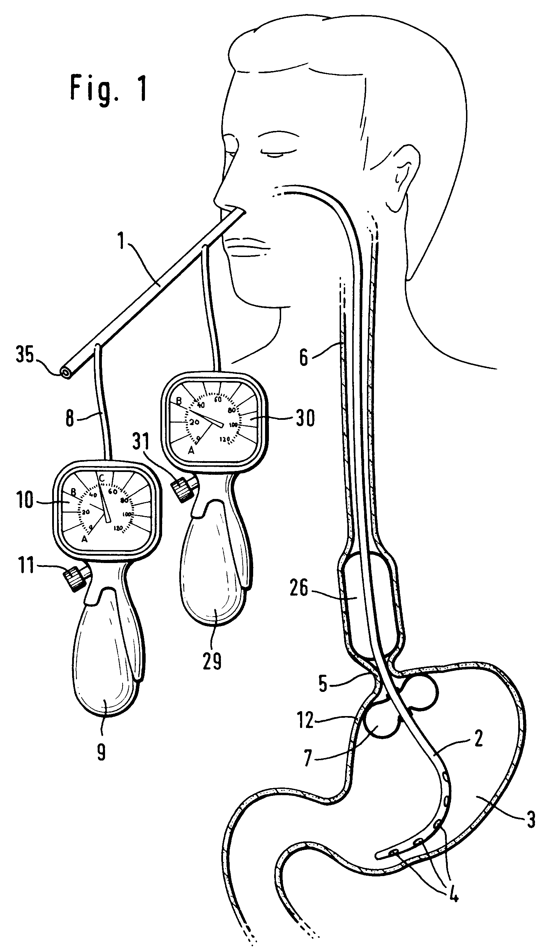

- Fig.1: eine Gesamtansicht und
- Fig.2: einen schematischen Längsschnitt durch den Magenballon.

Die Magensonde umfaßt einen Saugschlauch 1, der in seinem Endabschnitt 2, der frei im Magen 3 liegen soll, Saugöffnungen 4 besitzt und mit seinem freien Anschlußende 35 in bekannter Weise an einen Saugapparat angeschlossen werden kann, um Mageninhalt abzusaugen. Zum Abdichten des Mageneingangs 5, bei welchem der untere Schließmuskel der Speiseröhre 6 eine Verengung bildet, dient ein Magenballon 7, dessen Innenraum über ein am Saugschlauch vorgesehenes, besonderes Lumen mit dem Anschußschlauch 8 verbunden ist, an den eine Handpumpe 9 mit Manometer 10 und Druckablaßschraube 11 angeschlossen ist. Mit dem Abschnitt 2 des Saugschlauchs 1 wird der Magenballon 7 im ungeblähten Zustand in den Magen eingeführt, dann mittels der Handpumpe 9 mit Rückschlagventil vom Druck Null (Druckstufe A) auf geringen Druck von beispielsweise 20-30 mbar (Druckstufe B) gebläht. Danach sind die Luftwege, die zu einem Auslaß der Luft aus dem Ballon führen könnten, geschlossen. Insbesondere ist dabei und bleibt anschließend die Entlüftungsschraube 11 geschlossen. Durch Zug am Saugschlauch 1 wird dieser gespannt, wobei sich der Magenballon 7 gegen die den Mageneingang 5 umgebende Magenwand 12 unter Ausübung eines Anlagedrucks anlegt, der maximal so groß ist wie der Innendruck im Magenballon 7. Dieser Innendruck ist um so größer, je stärker sich der Magenballon 7 unter dem Zug des Saugschlauchs 1 an die Magenwand anlegt. Er wird mittels des Manometers 10 kontrolliert und soll als Druckstufe C etwa 10-20 mbar höher liegen als die Druckstufe B. Der Saugschlauch 1 wird in seiner Lage fixiert, sobald dieser Druck erreicht ist. Soweit sind die Vorrichtung und ihre Verwendung bei dem Stand der Technik, von dem die Erfindung ausgeht, bekannt.

Der Magenballon 7 hat eine Form, die sich der eines Torus annähert. Sie kommt dadurch zustande, daß die Stellen 13, 14, an denen die Wände 15, 16 des Magenballons 7 an dem Saugschlauch 1 befestigt sind, einen geringen Abstand 17 voneinander haben. Dieser Abstand beträgt beispielsweise 12mm bei einem Magenballon mit einem Außendurchmesser von 6cm. Er ist jedenfalls geringer als die Radialabmessung 18 und die parallel zum Saugschlauch gemessene Abmessung 19 des Ballons. Sie kann noch geringer sein als es in dem Beispiel gezeigt ist und beispielsweise bis auf Null schrumpfen. Als Befestigungsstellen sind diejenigen anzusehen, an denen sich die den Ballon bildende Membran vom Saugschlauch 1 unter Bildung der Ballonwand abhebt.

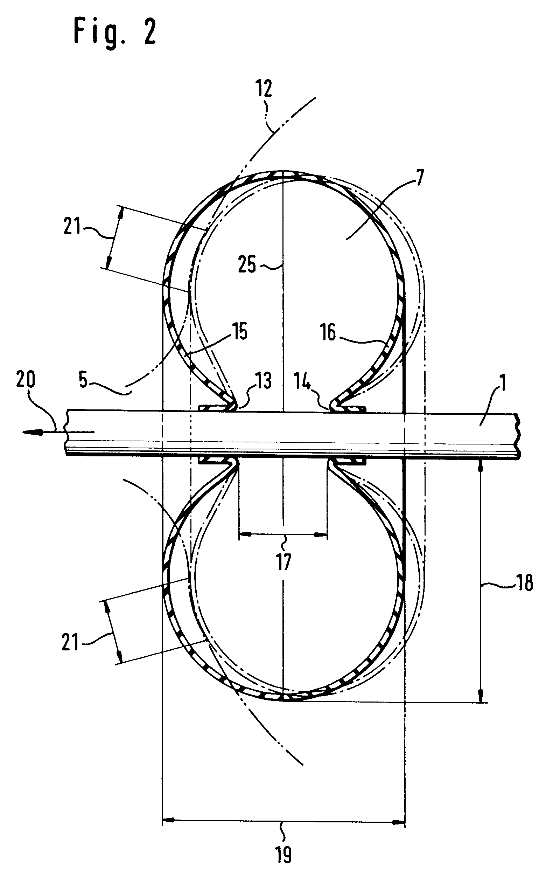

Im entspannten, geblähten Zustand hat der Magenballon die in Fig.2 mit durchgezogenen Linien dargestellte Längsschnittgestalt. Wirkt auf ihn unter dem Zug 20 des Saugschlauchs 1 die Anlagekraft der Magenwand 12, so verformt er sich in dem in Fig.2 strichpunktiert angedeuteten Sinn, wobei er eine bemerkenswerte Elastizität in Längsrichtung des Schlauchs zeigt. Dabei kommt hauptsächlich eine Ringfläche 21, die in Fig.2 mit ihrer Radialabmessung bezeichnet ist, an der Magenwand 12 zur Anlage. Der größte in dieser Anlagefläche erreichte Druck gleicht dem Innendruck des Magenballons. In dem Bereich, der neben und radial innerhalb dieser Ringfläche liegt, findet keine Anlage statt oder - falls sie stattfindet - ist der darin herrschende Anlagedruck vernachlässigbar gering. Die Größe der Fläche, innerhalb welcher der Balloninnendruck als Anlagedruck auf die Magenwand wirkt, ist demzufolge sehr gering. Entsprechend gering ist auch die im Saugschlauch 1 erforderliche Zugkraft 20. Dennoch kommt eine hinreichende Dichtwirkung zustande, weil die Ringfläche 21 den Mageneingang zuverlässig umschließt und darin auch mit größerer Sicherheit als bei großflächiger Anlage ringsum der für eine gute Dichtwirkung erforderliche Anlagedruck erreicht wird.

Anders als bei einem kugelförmigen Ballon verläuft diese Anlagefläche nahezu lotrecht zur Richtung des Saugschlauchs 1 und übt deshalb praktisch keine aufweitende Keilwirkung auf den Mageneingang aus. Der erfindungsgemäße Magenballon kann deshalb aus zwei Gründen mit wesentlich kleinerem Durchmesser als herkömmliche, kugelförmige Magenballons ausgeführt werden, weil zum einen der Zug im Saugschlauch geringer ist und weil zum anderen die von dem Ballon auf den Mageneingang ausgeübte, öffnende Keilwirkung geringer ist. Dies hat wiederum eine Verringerung der Anlagefläche zur Folge, was sich günstig auf die Höhe der erforderlichen Saugschlauchspannung auswirkt.

Der Magenballon 7 ist aus zwei ebenen, runden Membranzuschnitten gefertigt, die an ihrem Außenumfang, der in dem geblähten Ballon gemäß Fig.2 als Äquatoriallinie 25 erscheint, miteinander verschweißt sind.

Die erfindungsgemäß erreichte, geringe Saugschlauchspannung wird vom Patienten in jedem Fall als angenehm empfunden, auch wenn der Saugschlauch mit einem Nasenstopper fixiert wird. Überdies gibt die geringe Saugschlauchspannung in vielen Fällen die Möglichkeit, die Fixierung mit einem Speiseröhrenballon 26 vorzunehmen. Dazu genügt oftmals schon die zwischen dem Speiseröhrenballon 26 und der Speiseröhrenwandung herrschende, geringe Reibung. Zweckmäßigerweise wird der Speiseröhrenballon statt dessen oder zusätzlich so angeordnet, daß sich sein unteres Ende an der Verengung der Speiseröhre abstützt, die durch deren unteren Schließmuskel am Mageneingang 5 gebildet ist. Der Abstand zwischen den Ballons 7 und 26 ist unter Berücksichtigung durchschnittlicher anatomischer Abmessungen so bemessen, daß diese Abstützung wirksam zustande kommt. Individuelle Maßunterschiede können durch die hohe Elastizität des Magenballons ausgeglichen werden. Die Oberfläche des Speiseröhrenballons 26 kann reibungsfördernd strukturiert sein, beispielsweise mit einer Vielzahl von Erhöhungen und Vertiefungen. Ferner ist er zweckmäßigerweise im geblähten Zustand zylindrisch, wobei sein Durchmesser so gewählt ist, daß sich durch die Dehnung der Speiseröhre die gewünschte Anlagespannung und Reibung ergibt. Zum Blähen des Speiseröhrenballons und zur Kontrolle seines Zustands kann eine weitere Handpumpe 29 mit Manometer 30, Rückschlagventil und Entlüftungsschraube 31 vorgesehen sein.

## Patentansprüche

1. Magensonde mit einem Saugschlauch (1) zum Ausleiten von Mageninhalt, einem Magenballon (7), dessen beide Wände (15,16) fest mit dem Saugschlauch (1) verbunden sind und der durch Spannung (20) des Saugschlauchs (1) in Anlage an der den Mageneingang (5) umgebenden Magenwand (12) zu halten ist, sowie mit einem den Saugschlauch (1) auf Spannung haltenden Stopper, wobei der Magenballon (7) mit einem Druckkontrollgerät (10) verbunden ist, das zur Anzeige von mindestens zwei Druckstufen (B,C) eingerichtet ist, von denen die niedrigere (B) dem bei ungespanntem Schlauch (1) frei geblähten Zustand des Magenballons (7) und die höhere (C) dem gespannten Zustand des Saugschlauchs (1) zugeordnet ist, dadurch gekennzeichnet, daß der Abstand (17) der Befestigungsstellen (13,14) der beiden Magenballonwände (15,16) am Schlauch (1) geringer ist als die radiale Ballonabmessung zwischen ihrem Außenumfang und der Schlauchoberfläche im geblähten Zustand bei der höheren Stufe (C) des Verwendungsdrucks, und daß der Magenballon in Längsrichtung des Schlauchs elastisch nachgiebig ist.

2. Magensonde nach Anspruch 1, dadurch gekennzeichnet, daß der Abstand (17) der Befestigungsstellen (13,14) nicht größer ist als zwei Drittel der Radialabmessung (18) des Magenballons (7).

3. Magensonde nach Anspruch 1 oder 2, dadurch gekennzeichnet, daß der Abstand (17) der Befestigungsstellen (13,14) geringer ist als die größte Ausdehnung (19) des Magenballons (7) in Richtung parallel zu dem diesen durchsetzenden Saugschlauchabschnitt.

4. Magensonde nach Anspruch 3, dadurch gekennzeichnet, daß der Abstand (17) der Befestigungsstellen (13,14) nicht größer ist als zwei Drittel der größten Ausdehnung (19) des Magenballons (7) in Richtung parallel zu dem ihn durchsetzenden Saugschlauchabschnitt.

5. Magensonde nach einem der Ansprüche 1 bis 4, dadurch gekennzeichnet, daß der Magenballon aus zwei im entspannten Zustand im wesentlichen ebenen Folienzuschnitten besteht, die am Rand (25) miteinander verschweißt sind und jeweils mittig von dem Schlauch (1) durchsetzt sind.

6. Magensonde nach einem der Ansprüche 1 bis 5, dadurch gekennzeichnet, daß der Stopper einen Speiseröhrenballon (26) umfaßt.

7. Magensonde nach Anspruch 6, dadurch gekennzeichnet, daß der Abstand zwischen dem Magenballon (7) und dem Speiseröhrenballon (26) so bemessen ist, daß der Speiseröhrenballon (26) bei gespanntem Saugschlauch (1) sich an der Oberseite des unteren Speiseröhrenschließmuskels anlegt.
